# EUROPEAN PATENT SPECIFICATION

(11) **EP 2 384 431 B1**
(45) Date of publication and mention of the grant of the patent: **29.11.2017**
(21) Application number: 09787366.5
(22) Date of filing: 06.10.2009
(51) Int. Cl.: G01N 21/47, G01N 21/49, G01N 21/59, A61B 5/00

(54) **DEVICE AND METHOD FOR OPTICALLY EXAMINING A TURBID MEDIUM COMPRISING JOINTS**
VORRICHTUNG UND VERFAHREN ZUR OPTISCHEN UNTERSUCHUNG EINES TRÜBEN MEDIUMS MIT GELENKEN
DISPOSITIF ET PROCÉDÉ POUR EXAMINER OPTIQUEMENT D'UN MILIEU TURBIDE COMPORTANT DES ARTICULATIONS

(30) Priority: 13.10.2008 EP 08166411
(43) Date of publication of application: 09.11.2011
(73) Proprietor: Hemics B.V., 5617 BC Eindhoven (NL)
(72) Inventor: VAN BEEK, Michael, C., NL-5656 AE Eindhoven (NL); RENSEN, Wouter, H., J., NL-5656 AE Eindhoven (NL); HARBERS, Rik, NL-5656 AE Eindhoven (NL)
(74) Representative: Nederlandsch Octrooibureau
(86) International application number: PCT/IB2009/054365
(87) International publication number: WO 2010/044003

(56) References cited:
- WO-A-99/04683
- WO-A-99/04684
- WO-A-2007/107909
- DE-A1- 10 004 989
- US-A1- 2011 066 034
- BEUTHAN J ET AL: "OPTISCHE DIFFUSIONSTOMOGRAPHIE ZUR MESSUNG OPTISCHER GEWEBEPARAMETER IN DER RHEUMADIAGNOSTIK. ÖOPTICA DIFFUSION TOMOGRAPHY FOR MEASUREMENT OF OPTICAL TISSUE PARAMETERS IN DIAGNOSIS OF RHEUMATISM" TECHNISCHES MESSEN TM, R.OLDENBOURG VERLAG. MUNCHEN, DE, vol. 63, no. 6, 1 June 1996 (1996-06-01), pages 234-240, XP000620446 ISSN: 0171-8096
- Multiple Authors: "Facial recognition system", Wikipedia, 30 September 2008 (2008-09-30), XP055320469, Retrieved from the Internet: URL:https://en.wikipedia.org/w/index.php?t itle=Special:Book&bookcmd=download&collect ion_id=d18a26b3f22a4ccfb7e91ad51e72f0c2651 e0427&writer=rdf2latex&return_to=Facial+re cognition+system [retrieved on 2016-11-17]
- Yong Xu ET AL: "Imaging of in vitro and in vivo bones and joints with continuous-wave diffuse optical tomography References and links", Optics Express, vol. 8, no. 7, 26 March 2001 (2001-03-26), pages 447-451, XP055374269,

## Description

### FIELD OF INVENTION

The present invention relates to a device for optically examining a turbid medium comprising joints and to a method for optically examining a turbid medium comprising joints.

### BACKGROUND OF THE INVENTION

In the context of the present application, the term light is to be understood to mean non-ionizing electromagnetic radiation, in particular with wavelengths in the range between 400 nm and 1400 nm. The term optically examining means examining by means of light. The term body part means a part of a human or animal body.

In recent years, several different types of devices for optically examining the interior of turbid media have been developed in which the turbid medium under examination, such as a body part, is illuminated with light from a light source and light emanating from the turbid medium is detected by a detector unit in transmission or reflection geometry. In such devices, the detected light is used to gather information about the interior of the turbid medium. Depending on the type of device for optically examining the interior of a turbid medium, e.g. two-dimensional or three-dimensional images of the interior of the turbid medium can be acquired or information about concentrations of different substances inside the turbid medium can be extracted from the detected light.

US 5 415 655 shows a medical device for examining tissue by means of light. The medical device has a flexible light guide having a light energy input end adapted for connecting to a light energy source and a light energy output end. The light energy output end outputs a beam of light energy.

WO 2007/107909 A1 discloses a device for imaging a turbid medium comprising: means for optically scanning a predefined maximum area of a scanning plane for acquisition of imaging data, means for detection of an outer contour of the turbid medium, and means for controlling the optical scanning such that a sub-area of the maximum area is scanned that is smaller than the maximum area and that covers the outer contour.

WO 99/04684 discloses a method of determining an examination point for a diaphanoscopic examination. Recently, it has been suggested to use devices for optically examining the interior of turbid media to optically detect disease activity of joint diseases such as rheumatoid arthritis (RA) by illuminating both the joints and intermediate tissue of a body part under examination with light and detecting light emanating from the body part. The treatment of such joint diseases is staged. Usually, a patient first receives pain killers. These are frequently followed by non-steroid anti-inflammatory drugs (NSAIDs) and disease modifying anti-rheumatic drugs (DMARDs). In many cases, the last stage in treatment with drugs is the use of biological therapies. In particular the last category is expensive and treatment can cost tens of thousands of dollars per year per patient. Additionally, the drugs used in later stages of treatment often cause more severe side effects. With respect to such joint diseases, medical professionals base their decisions on changes in therapy on disease activity which is given by the number and the severity of inflamed joints.

Since rheumatoid arthritis is a progressive disease and early diagnosis and start of treatment can help postponing adverse effects and high costs of treatment, there is a demand for methods and devices for providing satisfactory information about the condition of joints and which assist a medical professional to come to a conclusion with respect to the actual joint condition. Conventionally, rheumatologists use the so-called Disease Activity Score (DAS-28) for diagnosis and treatment monitoring. Since this method is time-consuming, operator-dependent, and has limited sensitivity, there is a demand for suitable devices for detecting disease activity. Use of devices for examining the respective body parts by means of light shows promising results as disease activity monitors.

According to a device for optically examining the interior of turbid media known to the applicant which device is specifically adapted for detecting disease activity of joint diseases, a turbid medium formed by a body part containing at least one joint, such as a human hand, is placed on a plate made of a transparent material. For examination, the turbid medium is illuminated with an extended light source positioned below the plate and, in transmission geometry, light is detected by a detector unit being a part of an imaging device situated on the opposite side of the turbid medium with respect to the light source. For example, the imaging device may be formed by a CCD camera acquiring a two-dimensional image of the turbid medium (or rather of the light transmitted through the turbid medium. However, in such an arrangement, e.g. in a case in which the turbid medium is a hand which is a typical situation for joint disease activity monitoring, light used for illuminating the turbid medium will also be transmitted from the light source to the detector unit without passing through the turbid medium. For example, the light will be transmitted between the fingers in the case of the turbid medium being formed by a hand. Since such light will not have been attenuated in the turbid medium, the intensity on the detector unit of this part of the light will be high as compared to the other part of the light which has passed through the turbid medium. Thus, the light not having passed through the turbid medium can saturate the detector unit such that, as a result, the relevant light which has passed through the turbid medium can only be detected with less accuracy.

### SUMMARY OF THE INVENTION

It is an object of the present invention to provide a device and a method for optically examining the interior of a turbid medium with which overexposure of a detector unit of an imaging device can be reliably prevented and the accuracy of examination of the interior of a turbid medium is increased.

This object is solved by a device for optically examining the interior of a turbid medium according to claim 1. The device comprises: an illumination system adapted for illuminating a turbid medium to be examined; and an imaging device adapted for generating images from detected light. The illumination system is adapted to be operable in at least: a first mode in which a large area is illuminated and a second mode in which at least one selected region of the large area is illuminated. Since the illumination system is operable in the two modes, first a wide area image can be acquired by illuminating the turbid medium under examination (and a surrounding area). From this wide area image, the region or regions of interest which are of particular interest and at which the turbid medium is actually situated can be determined. Then, these regions of interest can be illuminated in the second mode. Thus, for illumination in the second mode, it is ensured that only such regions are illuminated in which the turbid medium is located and all the light arriving at the imaging device will have been transmitted through or reflected by the turbid medium (depending on the arrangement). Thus, no light which has not been coupled to the turbid medium and which might cause overexposure of the detector unit of the imaging device will reach the imaging device. The device comprises a control unit adapted to analyze a first image acquired by the imaging device with the illumination system in the first mode and to determine the at least one selected region for illumination in the second mode from the first image. Thus, the selected region to be illuminated for the actual examination can be determined in an automated way. The selected region can e.g. be determined by an automated image analysis function. For example, in the case of the turbid medium being a human hand, such as in typical applications for joint disease monitoring, when illuminating the turbid medium in the first mode, light illuminating the support in the region of the turbid medium will be attenuated rather strongly while light illuminating the support next to the hand will reach the detector unit of the imaging device at least substantially un-attenuated. Thus, the position of the turbid medium will be a shadow in the first image which can easily be recognized such that the selected regions for illuminating in the second mode, such as joints and intermediate tissue, can be reliably identified. A plurality of selected regions of the large area is illuminated in the second mode. The plurality of selected regions comprises the positions of joints and intermediate tissue for examining the condition of joints.

According to one aspect, the illumination system is adapted to operate at low output light levels in the first mode and to operate at high output light levels in the second mode. In the first mode, only the position of the turbid medium has to be determined and un-attenuated light will reach the detector unit of the imaging device. By using low output light levels in the first mode, overexposure of the detector unit is prevented. In the second mode, there is no remaining risk of overexposure, since only the regions of interest are illuminated. Thus, high output light levels suitable for achieving high image quality are used. According to another aspect, the imaging system is adapted to acquire images with short integration times, when the illumination system operates in the first mode, and to acquire images with longer integration times, when the illumination system operates in the second mode. In this case, overexposure is prevented without requiring different output light levels such that a less expensive illumination system can be used. High image quality in the second mode is achieved by exploiting longer integration times.

According to an aspect, the illumination system comprises a pixilated light source unit for generating light for illuminating in the first and second modes. In this case, the illumination system can easily switch between the first mode and the second mode, by activating a large number of pixels (or even all) in the first mode and activating only selected pixels in the second mode. In this case, the illumination system can e.g. be formed by a two-dimensional matrix of LEDs (light emitting diodes) or by another kind of emissive display technology enabling creation of a pixilated illumination pattern.

According to another aspect, the illumination system comprises an extended light source unit and a spatial filter unit for generating light for illuminating in the first and second modes. In this case, a cost-efficient light source can be used and the first and second modes can be realized by a spatial filter such as a pixilated liquid crystal device. For example, one or more lamps, e.g. emitting in the IR (infrared), could be used as a light source. The transmission of the individual liquid crystal pixels can be used to achieve a desired illumination pattern in the second mode. In this case, preferably the spatial filter unit is arranged such that it is at close distance to the turbid medium to be examined and thus at close distance to the support.

According to still another aspect, the illumination system is adapted for projecting desired illumination patterns directly on the turbid medium. Possible realizations for such illumination systems include a light source in combination with a spatial light modulator (SLM) such as a light source in combination with a digital mirror device (such as a digitally controlled micro-mirror) or a light source in combination with a liquid crystal cell. Alternatively, a possible realization is a scanning laser system in which a laser beam is scanned over the support/turbid medium by a scanner.

According to a still further aspect, the illumination system comprises a plurality of individual illumination devices the positions of which are adjustable. For example, in a device for examining a human hand (e.g. a device for monitoring joint disease activity) a plurality of individual light sources for different fingers and for the wrist may be provided. Due to the adjustability of the positions, the illumination pattern for the second mode can be adapted to the shape of the actual hand to be examined.

Preferably, the device comprises a support which is adapted to support a body part comprising at least one joint as a turbid medium to be examined. In this case, the device is particularly suited for examining the condition of joints such as with respect to rheumatic arthritis. For example, the support may be adapted to accommodate a human hand as a turbid medium to be examined.

Preferably, the device is a medical optical examination apparatus. Even more preferably, the device is a device for optical examination of joints. In this case, the condition of joints can be reliably and conveniently examined.

The object is also solved by a method for optically examining the interior of a turbid medium according to claim 13. The method comprises the steps: illuminating a turbid medium to be examined over a large area and detecting light as a first image, determining at least one selected region of the large area from the first image, and illuminating the at least one selected region and detecting light as a second image. Thus, the advantages described above with respect to the device can be achieved. For acquisition of the second image, only one or more selected regions of the large area are illuminated. Since the at least one selected region is determined based on the first image, all light in the illumination pattern of the selected region(s) is reliably coupled to the turbid medium and light which has not been coupled to the turbid medium will not arrive at the detector unit of the imaging device. Thus, overexposure is reliably prevented and high accuracy examination of the interior of the turbid medium is enabled.

Preferably, illuminating over the large area and illuminating the at least one selected region is performed with the same illumination system and image acquisition of the first and second images is performed with the same imaging device. In this case, no additional parts (additional to the components for performing the actual measurement) are required and the alignment of the turbid medium is easily achieved.

### BRIEF DESCRIPTION OF THE DRAWINGS

Further features and advantages of the present invention will arise from the detailed description of embodiments with reference to the enclosed drawings.
Fig. 1 schematically shows the general set-up of a device for examining the interior of a turbid medium.
Fig. 2 schematically shows a desired illumination pattern for the case of optically examining the joint condition of human hands.
Fig. 3 schematically shows a first embodiment.
Fig. 4 schematically shows a second embodiment.
Fig. 5 schematically shows a third embodiment.
Fig. 6 schematically shows a fourth embodiment.
Fig. 7a schematically shows a fifth embodiment in a side view.
Fig. 7b schematically shows the illumination system of the fifth embodiment in top view.

### DETAILED DESCRIPTION OF EMBODIMENTS

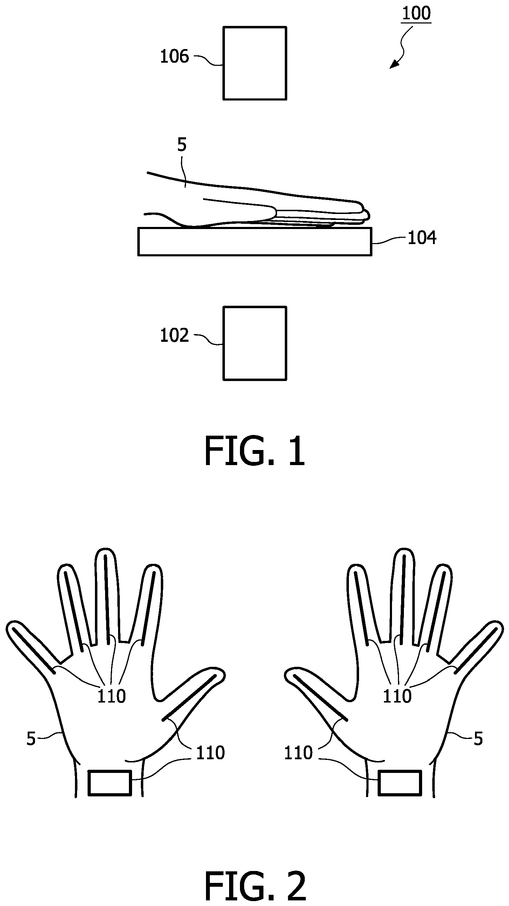

Embodiments will now be described with reference to the drawings. First, the general set-up of a device for optically examining the interior of a turbid medium which is adapted for examination of joint condition will be explained with respect to Fig. 1.

As can be seen in Fig. 1, the device for optically examining the interior of a turbid medium 100 comprises a support 104 which is adapted to support a turbid medium 5 to be examined. In the example shown in Fig. 1, the device for optically examining the interior of a turbid medium 100 is a device for optical examination of joints, in particular for joint disease monitoring, and the turbid medium 5 is a human hand. For joint disease monitoring, joints and intermediate tissue are examined as will be described below.

In the example of Fig. 1, the support 104 is formed by a substantially flat transparent plate upon which the turbid medium 5 is placed for examination such that the turbid medium 5 is supported during examination. For example, the transparent plate can consist of transparent glass, transparent PMMA, or other suitable transparent materials. For example the plate can also be curved for improved comfort and contact of the turbid medium during the measurement.

On one side of the support 104, an illumination system 102 comprising at least one light source is provided for illuminating the turbid medium 5 during examination. In the example show, the illumination system 102 is positioned below the support 104 such that the turbid medium 5 is illuminated through the support 104. The illumination system 102 is adapted such that at least a substantial part of the support 104 at which a turbid medium 5 is placed during examination and thus of the turbid medium 5 to be examined is illuminated. For example, the light source of the illumination system 102 may be a broad-band light source, such as an incandescent lamp, or a single color light source, such as an LED or a laser.

On the opposite side of the support 104 with respect to the light source 102, an imaging device 106 adapted for generating images from detected light is provided. The imaging device 106 comprises a detector unit for detecting light emanating from the support 104 and the turbid medium 5, respectively, as a result of the support 104 and/or the turbid medium 5 being illuminated by the illumination system 102. The imaging device 106 comprises a plurality of detection elements arranged in a two-dimensional matrix such that a spatially resolved image can be acquired. For example, the imaging device 106 can be formed by a CCD camera, a CMOS camera, an IR camera, and the like.

Although an arrangement is shown in which the support 104 is arranged between the light source 102 and the turbid medium 5, an interchanged arrangement with the support 104 being arranged between the turbid medium 5 and the imaging device 106 is also possible.

In operation of the device for examining the interior of a turbid medium 100, a turbid medium 5 which is supported by the support 104 is illuminated by the illumination system 102 and an image of (at least parts of) the turbid medium 5 is acquired by the imaging device 106.

Fig. 2 shows human hands as an example for a turbid medium 5 to be examined. For optically examining the condition of joints, the regions containing joints are of particular interest. The black bars 110 shown in Fig. 2 indicate desired illumination patterns (regions of interest) for examining the condition of joints in a hand, namely regions covering the finger joints and regions covering the wrist.

As has already been described above, since the illumination system 102 illuminates the whole turbid medium 5, light from the light source which has not been transmitted through the turbid medium 5 will also arrive at the detector unit of the imaging device 106. Since it has not been attenuated by the turbid medium 5, this light will have a higher intensity as compared to light which has passed through the turbid medium 5. Thus, this light may cause overexposure of the detector unit of the illumination system 102 such that the accuracy of detected light from the regions of interest 110 is adversely affected. Further, it should be kept in mind that turbid media to be examined cannot easily be positioned in the device in an exactly predetermined manner that the shape of different turbid media (such as hands from different individuals) will be different.

Now, embodiments will be described which solve the above problem of un-attenuated light reaching the detector unit of the imaging device 106.

Since the general set-up of the embodiments corresponds to that described with reference to Fig. 1, only the differences will be described and description of corresponding features will not be repeated.

### FIRST EMBODIMENT

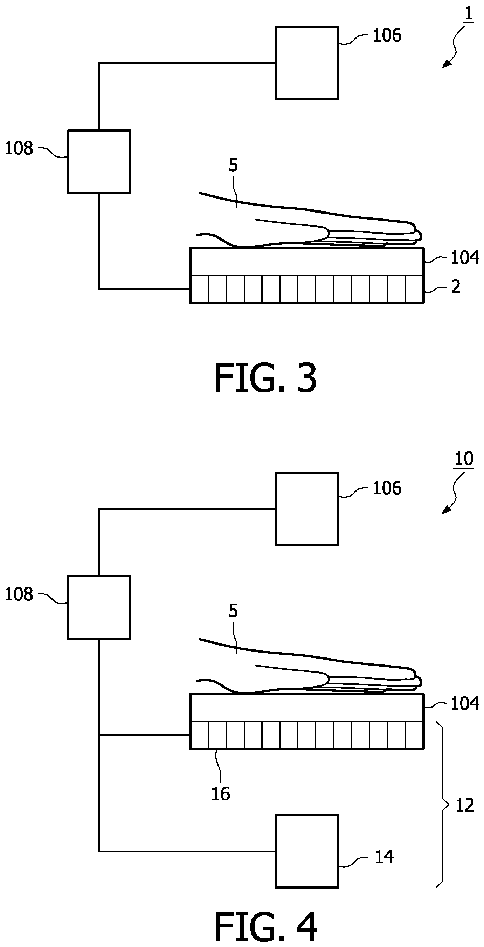

A first embodiment will be described with reference to Fig. 3. The device for optically examining the interior of turbid media 1 according to the first embodiment differs from the device explained with reference to Fig. 1 in the realization of the illumination system. According to the first embodiment, an illumination system 2 is provided. The illumination system 2 comprises a pixilated light source the individual pixels of which can be selectively activated to emit light. The pixilated light source is arranged in proximity to the turbid medium 5 to be examined such that desired illumination patterns for the turbid medium 5 to be examined can be realized by selectively activating pixels of the light source. For example, the pixilated light source can be formed by a two-dimensional matrix of LEDs (light emitting diodes). However, as an alternative other kinds of emissive display technologies allowing selectively emitting light from a plurality of pixels (arranged in a matrix) can be used to generate a pixilated pattern. A control unit 108 is provided to control operation of the device for examining the interior of a turbid medium 1. The control unit 108 is connected to the illumination system 2 and to the imaging device 106.

Now operation of the first embodiment will be described. In operation, the illumination device 2 is first controlled by the control unit 108 such that, in a first mode, a large area of the support 104 and thus a large area of the turbid medium 5 is illuminated. For example, the whole hand and an area surrounding the hand are illuminated in the first mode, in case of a device for examining joint condition. In this first mode, both light which has been transmitted through the turbid medium 5 and light which has passed next to the turbid medium 5 will reach the detector unit of the imaging device 106 such that a first image is formed. Thus, the turbid medium 5 (in particular its outline) can easily be detected as a shadow in the first image. As a consequence, a region which is of particular interest (ROI, region of interest) can be identified in the first image, since the position of the turbid medium 5 with respect to the support 104 can be determined. A plurality of such regions can be identified, e.g. the regions 110 described with respect to Fig. 2. Detection of the position of the turbid medium and determination of the regions 110 can e.g. be performed by an operator to which the first image is presented or in an automated way using known image analyzing techniques which can e.g. be implemented in the control unit 108, for instance as a software running in the control unit 108.

Thus, based on the first image acquired in the first mode, selected regions of interest 110 to be illuminated in a second mode are determined.

Then, the illumination device 2 is operated in a second mode in which only the selected regions 110 are illuminated. In the embodiment, this is achieved by activating only those pixels of the pixilated light source which correspond to the selected regions 110. With this set-up, a second image is acquired by the imaging device 106. Thus, in the second mode, only selected regions 110 are illuminated. These regions are selected such that light is emitted only where the turbid medium 5 is present. Thus, only light which has traveled through the turbid medium 5 will arrive at the detector unit of the imaging device 106 and there is no risk of overexposure during acquisition of the second image.

The position of selected regions of interested to be illuminated in the second mode is determined from the outline of a turbid medium to be examined.

In order to prevent overexposure of the detector unit of the imaging device 106 during acquisition of the first image, the illumination system 2 is adapted to output light on a low output level (low intensity) when operated in the first mode and to output light on a high output level (high intensity) when operated in the second mode. Thus, overexposure can be reliably prevented and high-accuracy examination is possible in the second mode. According to an alternative realization, the detector unit of the imaging device 106 can be operated with short integration times when the illumination system 2 is operated in the first mode and with longer integration times when the illumination system 2 is operated in the second mode. Operation of the device for examining the interior of a turbid medium with low light intensity in the first mode and with high light intensity in the second mode, or with different integration times is also possible for the further embodiments described below but will not be described in detail again.

### SECOND EMBODIMENT

A second embodiment will now be described with respect to Fig. 4. The device according to the second embodiment also differs in the realization of the illumination system. According to the second embodiment, an illumination system 12 is provided which comprises a spatially extended light source unit 14 and a spatial filter 16. The spatial filter 16 is positioned at close distance to the support 104 and thus to the turbid medium 5 to be examined. For example, the light source unit can be formed by one or more lamps, for instance lamps emitting light in the IR (infrared). The spatial filter 16 is adapted such that the light from the light source is selectively transmitted to the turbid medium 5 at selected positions only. To achieve this, the spatial filter 16 comprises a plurality of pixels arranged in a matrix which can be independently controlled to transmit or block the light from the light source unit 14. For example, the spatial filter 16 can be formed by a pixilated liquid crystal device. The second embodiment also comprises the control unit 108 connected to the illumination system 12 and to the imaging device 106 which is adapted to operate the device for examining the interior of a turbid medium in at least the first mode and the second mode.

However, as compared to the first embodiment, in the second embodiment illuminating a large area in the first mode and provision of a desired illumination pattern in the second mode is achieved by suitably operating the spatial filter 16. In the first mode, a large plurality of pixels of the spatial filter 16 is in a transmissive state. In the second mode, only selected pixels of the spatial filter 16 (only those corresponding to the desired illumination pattern) are controlled to be transmissive.

Thus, only the construction of the illumination system 12 and the technique to realize the first and second modes is different from the first embodiment and operation of the device such as determination of the selected region(s) is similar to that described above with respect to the first embodiment.

### THIRD EMBODIMENT

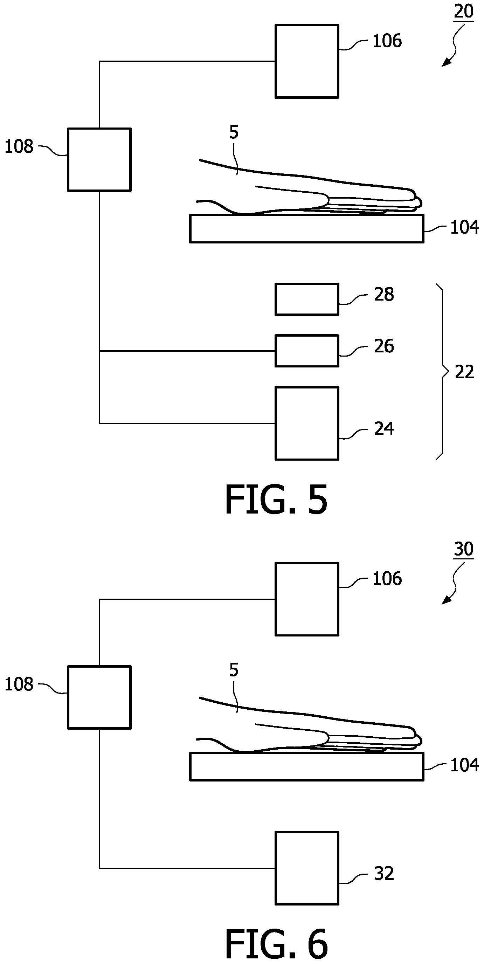

A third embodiment will now be described with respect to Fig. 5. The third embodiment only differs from the first embodiment in the way in which the first mode and the second mode for illumination (i.e. large area illumination and illumination of selected regions of interest only) are realized. Thus, only these differences will be described in the following. According to the third embodiment, the device for optically examining the interior of a turbid medium 20 comprises an illumination system 22. The illumination system 22 is adapted for projecting the desired illumination pattern directly on the turbid medium 5 to be examined.

The illumination system 22 of the third embodiment comprises light source unit 24, a spatial light modulator 26, and projection optics 28. For example, the spatial light modulator 26 can be formed by a digital mirror device or by a liquid crystal cell. The spatial light modulator 26 and the light source unit 24 are connected to the control unit 108. For realizing the second mode of operation (illumination of only selected regions), the illumination system 22 is controlled such that light is only projected to selected regions 110, while in the first mode the light from the light source unit 24 is projected to a larger area, e.g. to a whole hand and parts of the support 104 surrounding the hand. Determination of the selected regions is achieved similar to the first embodiment.

### FOURTH EMBODIMENT

A fourth embodiment will be described with respect to Fig. 6. The device for optically examining the interior of a turbid medium 30 according to the fourth embodiment corresponds to that of the third embodiment in that the illumination device 32 is adapted for projecting the desired illumination pattern directly on the turbid medium 5 to be examined. However, in contrast to the third embodiment, the illumination device 32 is a scanning illumination device, such as a laser scanner device, which subsequently directs a light beam to the turbid medium in the desired pattern. However, operation of the device for examining the interior of a turbid medium 30 according to the fourth embodiment is similar to the operation described with respect to the first to third embodiments. In the first mode, the scanning illumination device is operated such that a large area is illuminated, while in the second mode the scanning illumination device is operated such that only selected regions of the large area are illuminated.

Determination of the selected regions is achieved similar to the first embodiment.

### FIFTH EMBODIMENT

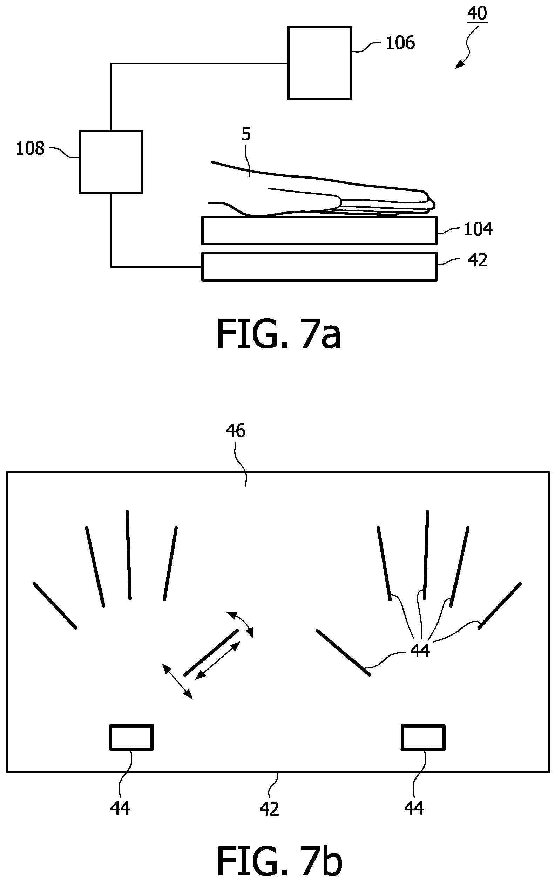

A fifth embodiment will be described with respect to Figs. 7a and 7b. Fig. 7a shows the device for optically examining the interior of a turbid medium 40 according the fifth embodiment in a side view. Fig. 7b is a schematic top view on the illumination system 42 of the device. The device of the fifth embodiment differs from the other embodiments in the construction of the illumination system only. In the following, only the differences to the other embodiments will be described.

The illumination system 42 of the fifth embodiment comprises a plurality of individual illumination devices 44. The individual illumination devices 44 are adapted to the shape of typical regions of interest for examination. In the embodiment shown in Fig. 7b, the shapes of the individual illumination devices 44 are adapted to the regions of interest of fingers and the wrist of a human hand. Thus, this embodiment is particularly adapted for examining the condition of joints. The individual illumination devices 44 are arranged in the illumination system 42 such that their position is individually adjustable in a plane parallel to the support 104 (e.g. by linear movements in two (perpendicular) directions in the plane and rotation about an axis perpendicular to the plane), as schematically indicated by arrows in Fig. 7b for one of the illumination devices 44. Such adjustability has the advantage that the position of the individual illumination devices 44 can be adapted to differences in shape between different turbid media, such as the differences in the position of fingers between different persons. Further, the illumination system 42 comprises an additional extended light source 46 adapted for illuminating a large area of the turbid medium 5 to be examined in the first mode.

As a result, according to the fifth embodiment in a first mode a large area is illuminated by the extended light source 46 and a first image is acquired by the imaging device 106. Then, similar to the other embodiments, the position of selected regions of interest is determined based on the first image. Then, according to the fifth embodiment the individual illumination devices 44 are actuated in the plane parallel to the support 104 to match the position of the turbid medium 5 which has been determined from the first image. In a second mode of operation, then only the selected regions of the large area are illuminated by the individual illumination devices 44 which have been moved to the correct positions.

Thus, according to the fifth embodiment as well, in the second mode of operation all light used for illumination is emitted only at positions where the turbid medium 5 is present. As a consequence, un-attenuated light will not reach the detector unit of the imaging device 106 such that overexposure and deterioration of the relevant images of regions of interest are prevented from occurring.

Thus, according to the embodiments described above, a turbid medium to be examined is, in a first mode, illuminated by large area illumination and the position of the turbid medium is determined from a first image acquired with this large area illumination. Regions of interest are selected based on the first image and the turbid medium is illuminated in a second mode in which only the selected regions are illuminated. A second image is acquired in the second mode. Thus, in both modes extended regions of the turbid medium under examination are illuminated and two-dimensional images of the turbid medium are acquired by the imaging device. Preferably, the same illumination system and imaging device are used for the first mode operation (determination of the regions of interest) and for the second mode of operation (examination of selected regions only). In this case, no additional parts are required and the alignment for the examination is easily achieved.

The proposed device for optically examining the interior of a turbid medium is particularly suited for optical detection of rheumatoid arthritis. It enables early and quantitative detection of inflammation. Thus, early treatment and treatment monitoring become possible. The proposed device is capable of illuminating body parts of interest in a correct way such that unwanted saturation of a detector unit is prevented.

## Claims

1. Device (1; 10; 20; 30; 40) for optical examination of a turbid medium (5) comprising joints, comprising:
an illumination system (2; 12; 22; 32; 42) adapted for illuminating the turbid medium (5) to be examined; and
an imaging device (106) adapted for generating images of the turbid medium (5) from detected light, which detected light is transmitted from the illumination system (2; 12; 22; 32; 42);
wherein the illumination system (2; 12; 22; 32; 42) is adapted to be operable in at least:
a first mode in which a first area is illuminated, the first area comprising the turbid medium (5) and a surrounding area, and
a second mode in which a plurality of selected regions (110) of the first area are illuminated;
wherein the device comprises a control unit (108) adapted to analyze a first image acquired by the imaging device (106) with the illumination system (2; 12; 22; 32; 42) in the first mode by recognizing a position of the turbid medium (5) as a shadow in the first image to determine the plurality of selected regions (110), in which the turbid medium (5) is situated, for illumination in the second mode,
wherein the plurality of selected regions (110) comprise positions of joints and intermediate tissue.

2. The device according to claim 1, wherein the illumination system (2; 12; 22; 32; 42) is adapted to operate at low output light levels in the first mode and to operate at high output light levels in the second mode.

3. The device according to any one of claims 1 to 2, wherein the imaging system (106) is adapted to acquire images with short integration times, when the illumination system (2; 12; 22; 32; 42) operates in the first mode, and to acquire images with longer integration times, when the illumination system operates in the second mode.

4. The device according to any one of claims 1 to 3, wherein the illumination system (2) comprises a pixilated light source unit for generating light for illuminating in the first and second modes.

5. Device according to any one of claims 1 to 3, wherein the illumination system (12) comprises an extended light source unit (14) and a spatial filter unit (16) for generating light for illuminating in the first and second modes.

6. Device according to any one of claims 1 to 3, wherein the illumination system (22; 32) is adapted for projecting desired illumination patterns directly on the turbid medium (5).

7. Device according to any one of claims 1 to 3, wherein the illumination system (42) comprises a plurality of individual illumination devices (44) the positions of which are adjustable.

8. Device according to any one of claims 1 to 7, wherein the device comprises a support (104) adapted to support a body part comprising the turbid medium (5) to be examined.

9. Device according to any one of claims 1 to 8, wherein the device is a medical optical examination apparatus.

10. Method for optical examination of a turbid medium (5) comprising joints, the method comprising the steps:
illuminating, by an illumination system operating in a first mode, the turbid medium (5) to be examined over a first area, the first area comprising the turbid medium (5) and a surrounding area, and detecting, by an imaging device (106), a first image of the turbid medium (5),
recognizing a position of the turbid medium (5) as a shadow in the first image using image analysis to determine a plurality of selected regions (110) of the first area, in which selected regions the turbid medium (5) is situated, wherein the plurality of selected regions (110) comprise positions of joints and intermediate tissue, and
illuminating, by the illumination system operating in a second mode, the plurality of selected regions (110) and detecting, by the imaging device (106), light as a second image.

11. Method according to claim 10, wherein illuminating over the first area and illuminating the at least one selected region (110) is performed with the same illumination system (2; 12; 22; 32; 42) and image acquisition of the first and second images is performed with the same imaging device (106).

## Patentansprüche

1. Vorrichtung (1; 10; 20; 30; 40) zur optischen Untersuchung eines trüben Mediums (5) mit Gelenken, umfassend:
ein Beleuchtungssystem (2; 12; 22; 32; 42), das zum Beleuchten des zu untersuchenden trüben Mediums (5) ausgelegt ist; und
eine Abbildungsvorrichtung (106), die zum Erzeugen von Bildern von dem trüben Medium (5) aus detektiertem Licht ausgelegt ist, wobei das detektierte Licht von dem Beleuchtungssystem (2; 12; 22; 32; 42) ausgesendet wird;
wobei das Beleuchtungssystem (2; 12; 22; 32; 42) so ausgelegt ist, dass es zumindest betreibbar ist in:
einer ersten Betriebsart, in der ein erster Bereich beleuchtet wird, wobei der erste Bereich das trübe Medium (5) und einen umgebenden Bereich umfasst, und
einer zweiten Betriebsart, in der eine Vielzahl ausgewählter Regionen (110) des ersten Bereichs beleuchtet werden;
wobei die Vorrichtung eine Steuereinheit (108) umfasst, die so ausgelegt ist, dass sie ein erstes Bild analysiert, das von der Abbildungsvorrichtung (106) aufgenommen wird, während sich das Beleuchtungssystem (2; 12; 22; 32; 42) in der ersten Betriebsart befindet, indem sie eine Position des trüben Mediums (5) als Schatten auf dem ersten Bild erkennt, damit sie die Vielzahl ausgewählter Regionen (110), in denen sich das trübe Medium (5) befindet, zum Beleuchten in der zweiten Betriebsart bestimmt,
wobei die Vielzahl ausgewählter Regionen (110) Positionen von Gelenken und Zwischengewebe umfasst.

2. Vorrichtung nach Anspruch 1, wobei das Beleuchtungssystem (2; 12; 22; 32; 42) so ausgelegt ist, dass es in der ersten Betriebsart bei niedrigen Ausgangslichtpegeln arbeitet und in der zweiten Betriebsart bei hohen Ausgangslichtpegeln arbeitet.

3. Vorrichtung nach Anspruch 1 oder 2, wobei das Abbildungssystem (106) so ausgelegt ist, dass es Bilder mit kurzen Integrationszeiten aufnimmt, wenn das Beleuchtungssystem (2; 12; 22; 32; 42) in der ersten Betriebsart arbeitet, und dass es Bilder mit längeren Integrationszeiten aufnimmt, wenn das Beleuchtungssystem in der zweiten Betriebsart arbeitet.

4. Vorrichtung nach einem der Ansprüche 1 bis 3, wobei das Beleuchtungssystem (2) eine Pixellichtquelleneinheit zum Erzeugen von Licht zum Beleuchten in der ersten und zweiten Betriebsart umfasst.

5. Vorrichtung nach einem der Ansprüche 1 bis 3, wobei das Beleuchtungssystem (12) eine diffuse Lichtquelleneinheit (14) und eine Raumfiltereinheit (16) zum Erzeugen von Licht zum Beleuchten in der ersten und zweiten Betriebsart umfasst.

6. Vorrichtung nach einem der Ansprüche 1 bis 3, wobei das Beleuchtungssystem (22; 32) so ausgelegt ist, dass es gewünschte Beleuchtungsmuster unmittelbar auf das trübe Medium (5) strahlt.

7. Vorrichtung nach einem der Ansprüche 1 bis 3, wobei das Beleuchtungssystem (42) eine Vielzahl von einzelnen Beleuchtungsvorrichtungen (44) umfasst, deren Position einstellbar ist.

8. Vorrichtung nach einem der Ansprüche 1 bis 7, wobei die Vorrichtung einen Träger (104) umfasst, der so ausgelegt ist, dass er ein Körperteil trägt, das das zu untersuchende trübe Medium (5) umfasst.

9. Vorrichtung nach einem der Ansprüche 1 bis 8, wobei die Vorrichtung ein medizinisches optisches Untersuchungsgerät ist.

10. Verfahren zur optischen Untersuchung eines trüben Mediums (5) mit Gelenken, wobei das Verfahren folgende Schritte umfasst:
Beleuchten, mit einem in einer ersten Betriebsart arbeitenden Beleuchtungssystem, des zu untersuchenden trüben Mediums (5) über einen ersten Bereich, wobei der erste Bereich das trübe Medium (5) und einen umgebenden Bereich umfasst, und Erfassen, mit einer Abbildungsvorrichtung (106), eines ersten Bilds des trüben Mediums (5),
Erkennen einer Position des trüben Mediums (5) als Schatten im ersten Bild unter Verwendung einer Bildanalyse zum Bestimmen einer Vielzahl ausgewählter Regionen (110) des ersten Bereichs, wobei sich in den ausgewählten Regionen das trübe Medium (5) befindet, wobei die Vielzahl ausgewählter Regionen (110) Positionen von Gelenken und Zwischengewebe umfasst, und
Beleuchten, mit dem in einer zweiten Betriebsart arbeitenden Beleuchtungssystem, der Vielzahl ausgewählter Regionen (110), und Detektieren, mit der Abbildungsvorrichtung (106), von Licht als zweites Bild.

11. Verfahren nach Anspruch 10, wobei das Beleuchten über den ersten Bereich und das Beleuchten der mindestens einen ausgewählten Region (110) mit demselben Beleuchtungssystem (2; 12; 22; 32; 42) erfolgen und die Bilderfassung des ersten und zweiten Bilds mit derselben Abbildungsvorrichtung (106) erfolgt.

## Revendications

1. Dispositif (1 ; 10 ; 20 ; 30 ; 40) pour l'examen optique d'un milieu turbide (5), comprenant des articulations, comprenant :
un système d'éclairage (2 ; 12 ; 22 ; 32 ; 42) conçu pour éclairer le milieu turbide (5) à examiner ; et
un dispositif d'imagerie (106) conçu pour générer des images du milieu turbide (5) à partir de la lumière détectée, cette lumière détectée étant transmise par le système d'éclairage (2 ; 12 ; 22 ; 32 ; 42) ;
le système d'éclairage (2 ; 12 ; 22 ; 32 ; 42) étant conçu pour fonctionner au moins :
dans un premier mode dans lequel une première zone est éclairée, la première zone comprenant le milieu turbide (5) et une zone environnante et
un deuxième mode dans lequel une pluralité de régions sélectionnées (110) de la première zone sont éclairées ;
le dispositif comprenant une unité de commande (108) conçue pour analyser une première image acquise par le dispositif d'imagerie (106) avec le système d'éclairage (2 ; 12 ; 22 ; 32 ; 42) dans le premier mode en reconnaissant une position du milieu turbide (5) sous la forme d'une ombre dans la première image afin de déterminer la pluralité de régions sélectionnées (110) dans lesquelles se trouve le milieu turbide (5), pour l'éclairage dans le deuxième mode,
la pluralité de régions sélectionnées (110) comprenant des positions d'articulations et du tissu intermédiaire.

2. Dispositif selon la revendication 1, dans lequel le système d'éclairage (2 ; 12 ; 22 ; 32 ; 42) est conçu pour fonctionner à des niveaux de sortie de lumière faibles dans le premier mode et pour fonctionner à des niveaux de lumière de sortie élevés dans le deuxième mode.

3. Dispositif selon l'une des revendications 1 à 2, dans lequel le système d'imagerie (106) est conçu pour acquérir des images avec des temps d'intégration courts, lorsque le système d'éclairage (2 ; 12 ; 22 ; 32 ; 42) fonctionne dans le premier mode et pour acquérir des images avec des temps d'intégration plus longs lorsque le système d'éclairage fonctionne dans la deuxième mode.

4. Dispositif selon l'une des revendications 1 à 3, dans lequel le système d'éclairage (2) comprend une unité de source lumineuse pixelisée pour l'éclairage dans les premier et deuxième modes.

5. Dispositif selon l'une des revendications 1 à 3, dans lequel le système d'éclairage (12) comprend une unité de source lumineuse étendue (14) et une unité de filtrage spatial (16) pour générer de la lumière pour l'éclairage dans les premier et deuxième modes.

6. Dispositif selon l'une des revendications 1 à 3, dans lequel le système d'éclairage (22 ; 32) est conçu pour projeter des motifs d'éclairage souhaités directement sur le milieu turbide (5).

7. Dispositif selon l'une des revendications 1 à 3, dans lequel le système d'éclairage (42) comprend une pluralité de dispositifs d'éclairage individuels (44) dont les positions sont ajustables.

8. Dispositif selon l'une des revendications 1 à 7, dans lequel le dispositif comprend un support (104) conçu pour supporter une partie de corps comprenant le milieu turbide (5) à examiner.

9. Dispositif selon l'une des revendications 1 à 8, dans lequel le dispositif est un appareil d'examen optique médical.

10. Procédé d'examen optique d'un milieu turbide (5) comprenant des articulations, ce procédé comprenant les étapes suivantes :
éclairage, à l'aide d'un système d'éclairage fonctionnant dans un premier mode, du milieu turbide (5) à examiner, sur une première zone, la première zone comprenant le milieu turbide (5), et une zone environnante, et détection, par un dispositif d'imagerie (106), d'une première image du milieu turbide (5),
reconnaissance d'une position du milieu turbide (5) sous la forme d'une ombre dans la première image à l'aide d'une analyse d'image afin de déterminer une pluralité de régions sélectionnées (110) de la première zone, le milieu turbide (5) se trouvant dans ces régions sélectionnées, la pluralité de régions sélectionnées (110) comprenant des positions d'articulations et du tissu intermédiaire et
éclairage, à l'aide du système d'éclairage fonctionnant dans un deuxième mode, de la pluralité de régions sélectionnées (110) et détection, par le dispositif d'imagerie (106), d'une lumière en tant que deuxième image.

11. Procédé selon la revendication 10, dans lequel l'éclairage sur la première zone et l'éclairage de l'au moins une région sélectionnée (110) sont effectués avec le même système d'éclairage (2 ; 12 ; 22 ; 32 ; 42) et l'acquisition de la première et de la deuxième images est effectuée avec le même dispositif d'imagerie (106).
